# EUROPÄISCHE PATENTANMELDUNG

(11) **EP 3 045 929 A1**
(43) Veröffentlichungstag der Anmeldung: **20.07.2016**
(21) Anmeldenummer: 15199836.6
(22) Anmeldetag: 14.12.2015
(51) Int. Cl.: G01R 33/28, G01R 33/563, G01R 33/34, G01R 33/36, G01R 33/565

(54) **SENSOR ZUR DETEKTION VON BEWEGUNGEN EINES PATIENTEN IN EINEM BILDGEBENDEN SYSTEM**

(30) Priorität: 15.01.2015 DE 102015200510
(71) Anmelder: Siemens Healthcare GmbH, 91052 Erlangen (DE)
(72) Erfinder: Nisznansky, Martin, 91096 Möhrendorf (DE); Rehner, Robert, 91077 Neunkirchen am Brand (DE); Vester, Markus, 90471 Nürnberg (DE)

(57) **Zusammenfassung**

Die Erfindung betrifft Verfahren und einen Bewegungssensor (AS) zur Detektion von Bewegungen (AB) eines Patienten (105) in einem bildgebenden medizinischen System, insbesondere in einem Magnetresonanztomographiesystem (101),
dadurch gekennzeichnet, dass er (AS) mindestens einen HF-Resonator (HF-Res) zum Senden eines von einer HF-Signal-Quelle (HF-Si-Srs) in ihn eingespeisten (CPL-in) HF-Signals (HF-Si) und zum Empfang (CPL-out, HF-Filt, HF-Dtect) eines Antwort-Signals (HF-Ressi) aufweist, und dass er (AS) eine Detektionseinrichtung (HF-Filt, HF-Detect) zur Detektion von Bewegungen (AB) des Patienten (105) aufweist.

## Beschreibung

Die Erfindung betrifft Bewegungssensoren und Verfahren zur Erfassung von Bewegungen eines Patienten in einem bildgebenden medizinischen System, insbesondere Magnetresonanztomographiesystem.

Verfahren zur Erfassung von Bewegungen (insbesondere zur Atemerfassung) in einem Magnetresonanztomographiesystem sind beispielsweise in folgenden Schriften beschrieben:
Die Veröffentlichung Buikman, Helzel, Röschmann: The Coil as a Sensitive Motion Detector for MRI-Magnetic-Resonance-Imaging, Vol. 6, Num. 3, 1988 der Firma Philips beschreibt, dass man durch die Messung des Reflexionsfaktors der Body-Coil eines MRT die Atembewegung detektieren kann.

DE 10 2014 209 488.7 von R. Rehner, A. Fackelmeier und S. Biber betrifft eine "Atemerfassung durch körpernahe posteriore Hochfrequenz-Spulen", wobei die Atmungsdetektion durch eine Messung des Reflexionsfaktors einer Sensor-Spule unter dem Patienten beschrieben ist.

DE 19 2009 052 412 A1 betrifft ein "Mess-System zur Erfassung der Lage eines sich bewegenden Organs".

Es ist eine Aufgabe der vorliegenden Erfindung, ein medizinisches Gerät (insbesondere Magnetresonanztomographiesystem) hinsichtlich einer Bewegungserfassung zu optimieren. Diese Aufgabe wird jeweils durch die Merkmale der unabhängigen Patentansprüche gelöst. Ausgestaltungen der Erfindung können eine zu bestehenden Varianten alternative, effiziente Bewegungserfassung ermöglichen. Vorteilhafte Weiterbildungen sind in den Unteransprüchen und der nachfolgenden Beschreibung angegeben.

Weitere Merkmale und Vorteile von möglichen Ausgestaltungen der Erfindung ergeben sich aus der nachfolgenden Beschreibung von Ausführungsbeispielen anhand der Zeichnung. Dabei zeigt:
- Fig. 1: ein Ausführungsbeispiel einer Messanordnung mit einem offenen LC-Resonator,
- Fig. 2: gemessene Änderungen der Transmission S21 durch eine Körperbewegung,
- Fig. 3: gemessene zeitliche Änderungen der Transmission S21 durch Atmung,
- Fig. 4: schematisch ein MRT-System.

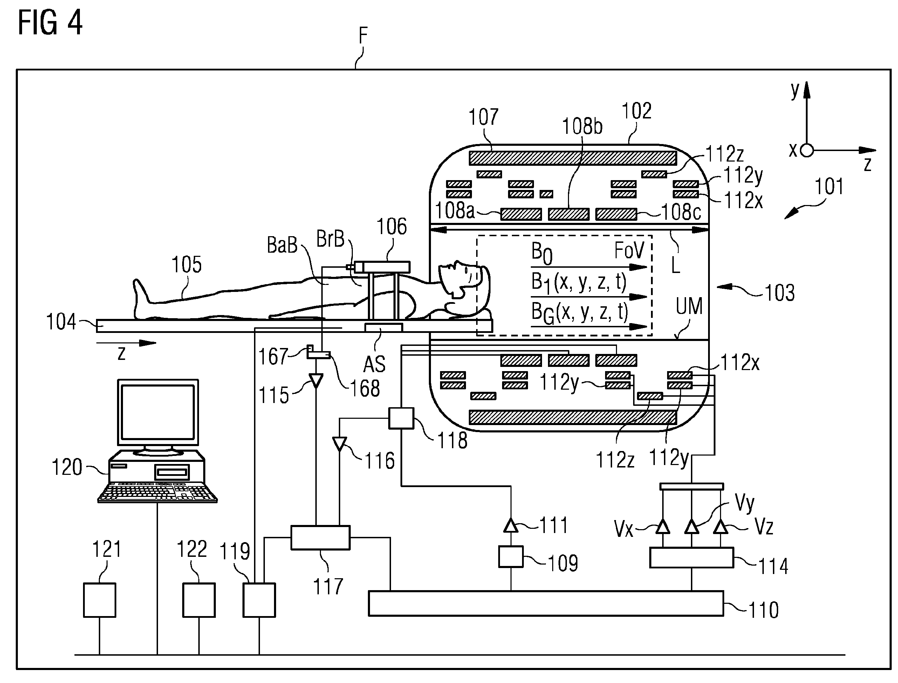

Figur 4 zeigt (u.a. insbesondere auch zum technischen Hintergrund) ein (in einem geschirmten Raum oder Faraday-Käfig F befindliches) bildgebendes Magnetresonanzgerät MRT 101 mit einem Hohlzylinder 102 mit einem hier röhrenförmigen Raum 103 in welchen eine Patientenliege 104 mit einem Körper z.B. eines Untersuchungsobjektes (z.B. eines Patienten) 105 (mit oder ohne Lokalspulenanordnung 106) in Richtung des Pfeils z gefahren werden kann, um durch ein bildgebendes Verfahren Aufnahmen des Patienten 105 zu generieren. Auf dem Patienten 105 ist hier eine Lokalspulenanordnung 106 angeordnet, mit welcher in einem lokalen Bereich (auch Field of View oder FoV genannt) des MRT-Aufnahmen von einem Teilbereich des Körpers 105 im FoV generiert werden können. Signale der Lokalspulenanordnung 106 können von einer z.B. über Koaxialkabel oder per Funk (167) etc. an die Lokalspulenanordnung 106 anschließbaren Auswerteeinrichtung (168, 115, 117, 119, 120, 121 usw.) des MRT 101 ausgewertet (z.B. in Bilder umgesetzt, gespeichert oder angezeigt) werden.

Um mit einem Magnetresonanzgerät MRT 101 einen Körper 105 (ein Untersuchungsobjekt oder einen Patienten) mittels einer Magnet-Resonanz-Bildgebung zu untersuchen, werden verschiedene, in ihrer zeitlichen und räumlichen Charakteristik genauestens aufeinander abgestimmte Magnetfelder auf den Körper 105 eingestrahlt. Ein starker Magnet (oft ein Kryomagnet 107) in einer Messkabine mit einer hier tunnelförmigen Öffnung 103, erzeugt ein statisches starkes Hauptmagnetfeld B₀, das z.B. 0,2 Tesla bis 3 Tesla oder auch mehr beträgt. Ein zu untersuchender Körper 105 wird auf einer Patientenliege 104 gelagert in einen im Betrachtungsbereich FoV (auch "Field Of View" oder "field of view" genannt) etwa homogenen Bereich des Hauptmagnetfeldes B0 gefahren. Eine Anregung der Kernspins von Atomkernen des Körpers 105 erfolgt über magnetische Hochfrequenz-Anregungspulse B1(x, y, z, t) die über eine hier als (z.B. mehrteilige = 108a, 108b, 108c) Körperspule 108 sehr vereinfacht dargestellte Hochfrequenzantenne (und/oder ggf. eine Lokalspulenanordnung) eingestrahlt werden. Hochfrequenz-Anregungspulse werden z.B. von einer Pulserzeugungseinheit 109 erzeugt, die von einer Pulssequenz-Steuerungseinheit 110 gesteuert wird. Nach einer Verstärkung durch einen Hochfrequenzverstärker 111 werden sie zur Hochfrequenzantenne 108 geleitet. Das hier gezeigte Hochfrequenzsystem ist lediglich schematisch angedeutet. Möglicherweise werden auch mehr als eine Pulserzeugungseinheit 109, mehr als ein Hochfrequenzverstärker 111 und mehrere Hochfrequenzantennen 108 a, b, c in einem Magnet-Resonanz-Gerät 101 eingesetzt.

Weiterhin verfügt das Magnet-Resonanz-Gerät 101 über Gradientenspulen 112x, 112 y, 112 z, mit denen bei einer Messung magnetische Gradientenfelder B_{G}(x, y, z, t) zur selektiven Schichtanregung und zur Ortskodierung des Messsignals eingestrahlt werden. Die Gradientenspulen 112x, 112y, 112z werden von einer Gradientenspulen-Steuerungseinheit 114 (und ggf. über Verstärker Vx, Vy, Vz) gesteuert, die ebenso wie die Pulserzeugungseinheit 109 mit der Pulssequenz-Steuerungseinheit 110 in Verbindung steht.

Von den angeregten Kernspins (der Atomkerne im Untersuchungsobjekt) ausgesendete Signale werden von der Körperspule 108 und/oder mindestens einer Lokalspulenanordnung 106 empfangen, durch zugeordnete Hochfrequenzvorverstärker 116 verstärkt und von einer Empfangseinheit 117 weiterverarbeitet und digitalisiert. Die aufgezeichneten Messdaten werden digitalisiert und als komplexe Zahlenwerte in einer k-Raum-Matrix abgelegt. Aus der mit Werten belegten k-Raum-Matrix ist mittels einer mehrdimensionalen Fourier-Transformation ein zugehöriges MR-Bild rekonstruierbar.

Für eine Spule, die sowohl im Sende- als auch im Empfangsmodus betrieben werden kann, wie z.B. die Körperspule 108 oder eine Lokalspule 106, wird die korrekte Signalweiterleitung durch eine vorgeschaltete Sende-Empfangs-Weiche 118 geregelt.

Eine Bildverarbeitungseinheit 119 erzeugt aus den Messdaten ein Bild, das über eine Bedienkonsole 120 einem Anwender dargestellt und/oder in einer Speichereinheit 121 gespeichert wird. Eine zentrale Rechnereinheit 122 steuert die einzelnen Anlagekomponenten.

In der MR-Tomographie werden Bilder mit hohem Signal/Rauschverhältnis (SNR) heute in der Regel mit so genannten Lokalspulenanordnungen (Coils, Local Coils) aufgenommen. Dies sind Antennensysteme, die in unmittelbarer Nähe auf (anterior) oder unter (posterior) oder an oder in dem Körper 105 angebracht werden. Bei einer MR-Messung induzieren die angeregten Kerne in den einzelnen Antennen der Lokalspule eine Spannung, die dann mit einem rauscharmen Vorverstärker (z.B. LNA, Preamp) verstärkt und schließlich an die Empfangselektronik weitergeleitet wird. Zur Verbesserung des Signal/Rauschverhältnisses auch bei hochaufgelösten Bildern werden so genannte Hochfeldanlagen eingesetzt (1.5T-12T oder mehr). Wenn an ein MR-Empfangssystem mehr Einzelantennen angeschlossen werden können, als Empfänger vorhanden sind, wird zwischen Empfangsantennen und Empfänger z.B. eine Schaltmatrix (teilweise auch RCCS genannt) eingebaut. Diese routet die momentan aktiven Empfangskanäle (meist die, die gerade im Field of View des Magneten liegen) auf die vorhandenen Empfänger. Dadurch ist es möglich, mehr Spulenelemente anzuschließen, als Empfänger vorhanden sind, da bei einer Ganzkörperabdeckung nur die Spulen ausgelesen werden müssen, die sich im FoV bzw. im Homogenitätsvolumen des Magneten befinden.

Als Lokalspulenanordnung 106 wird z.B. allgemein ein Antennensystem bezeichnet, das z.B. aus einem oder als Array-Spule aus mehreren Antennenelementen (insb. Spulenelementen) bestehen kann. Diese einzelnen Antennenelemente sind z.B. als Loopantennen (Loops), Butterfly, Flexspulen oder Sattelspulen ausgeführt. Eine Lokalspulenanordnung umfasst z.B. Spulenelemente, einen Vorverstärker, weitere Elektronik (Mantelwellensperren etc.), ein Gehäuse, Auflagen und meistens ein Kabel mit Stecker, durch den sie an die MRT-Anlage angeschlossen wird. Ein anlagenseitig angebrachte Empfänger 168 filtert und digitalisiert ein von einer Lokalspule 106 z.B. per Funk etc. empfangenes Signal und übergibt die Daten einer digitalen Signalverarbeitungseinrichtung die aus den durch eine Messung gewonnenen Daten meist ein Bild oder ein Spektrum ableitet und dem Nutzer z.B. zur nachfolgenden Diagnose durch ihn und/ oder Speicherung zur Verfügung stellt.

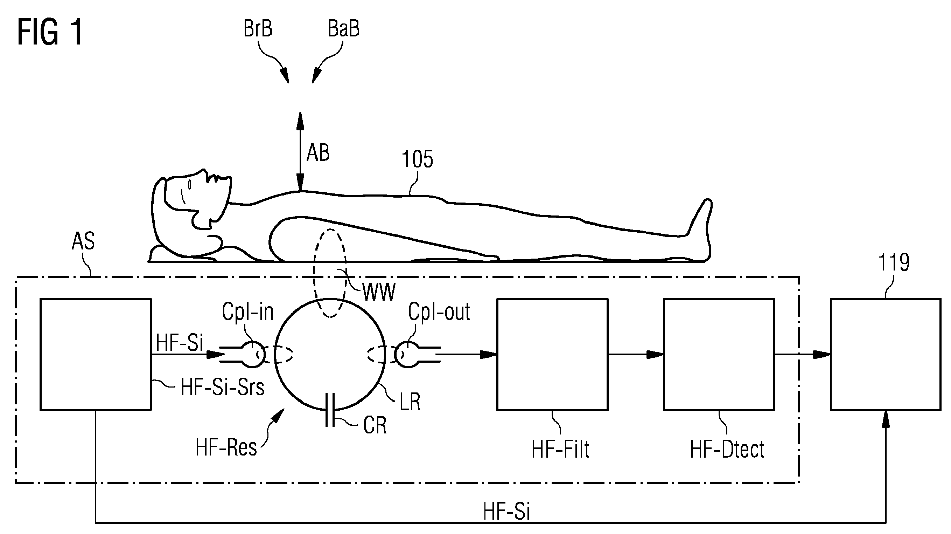

Fig. 1-4 zeigen einige Details beispielhafter Ausgestaltungen der Erfindung.

Bei der medizinischen Bildgebung können ungewünschte Patienten-Bewegung während der Bildaufnahme starke Bildartefakte verursachen. Solche ungewünschte Bewegungsartefakte können im Brust- oder Bauch-Bereich auch durch Atmung verursacht werden. Deshalb kann es sinnvoll sein, die Patienten-Atmung zu detektieren und die (MRT-)Bildaufnahme mit dem Atmung-Zyklus zu synchronisieren. Das kann insbesondere hilfreich sein für Magnetresonanztomographie (MRT), weil die Bildaufnahme (MR-Sequenz) einige Minuten dauern kann und nicht jeder Patient den Atem so lange anhalten kann.

Fig. 1 zeigt eine Ausgestaltung der Erfindung mit einer Platzierung hier eines oder alternativ mehrerer HF-Resonatoren (Schwingkreise) HF-Res in der Nähe des Patienten 105 im Brustbereich BrB und/oder Bauch-Bereich BaB. Der HF-Resonator HF-Res (und/oder der Bewegungssensor AS mit Auswertungselektronik) kann z.B. in die Patientenliege 104 eines Magnetresonanztomographiegeräts 101 integriert werden. In den HF-Resonator HF-Res wird ein HF-Signal aus einem HF-Generator (HF-Si-Srs) z.B. über eine Einkoppelungs-Spule Cpl-In (oder kapazitiv mit z.B. einem Koppelkondensator oder mit anderer Koppelung) eingekoppelt. Das in dem HF-Generator HF-Si-Srs erzeugte, in den HF-Resonator HF-Res eingekoppelte HF-Signal wird aus dem HF-Resonator wieder (induktiv oder kapazitiv mit z.B. einem Koppelkondensator oder mit anderer Koppelung) ausgekoppelt (über Cpl-out) und mit dem z.B. schmalbandigen HF-Detektor (HF-Filt und/oder HF-Dtect) gefiltert und detektiert.

Eigenschaften (WW, wie hier insbesondere Güte) des HF-Resonators werden hier durch die Atmung des Patienten beeinflusst und dadurch ändert sich auch die Transmission (S21) des HF-Signals über den HF-Resonator (also die Differenz S21diff zwischen S21-e beim Patienten der eingeatmet hat im Vergleich zu S21-a beim Patienten der ausgeatmet hat). Diese Anordnung ermöglicht eine Messung der Transmissionsverluste des HF-Resonators HF-Res, wie in Fig. 1 dargestellt ist.

Der HF-Resonator HF-Res ist hier so aufgebaut, dass das induzierte elektromagnetische Feld HF-Si des HF-Resonators HF-Res in den Körper des Patienten 105 eindringt damit eine Wechselwirkung WW zwischen dem HF-Resonator HF-Res und dem Körper des Patienten 105 ermöglicht wird. Eine Körperbewegung AB (wie auch z.B. eine Atembewegung oder eine Verschiebung der Anatomie innerhalb des Körpers, die nach außen auch nicht sichtbar sein könnte) verursacht dann hier eine Änderung der belasteten Güte des HF-Resonators HF-Res.

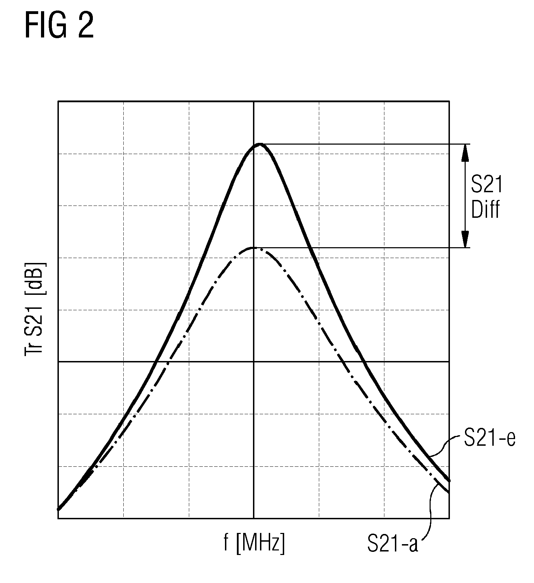

Das kann z.B. wie in Fig. 2 dargestellt als Änderung S21diff (als Differenz der Transmission S-21-e im eingeatmeten und S21-a im ausgeatmeten Zustand des Körpers des Patienten) der Transmission S21[dB] in Abhängigkeit von der Frequenz detektiert werden.

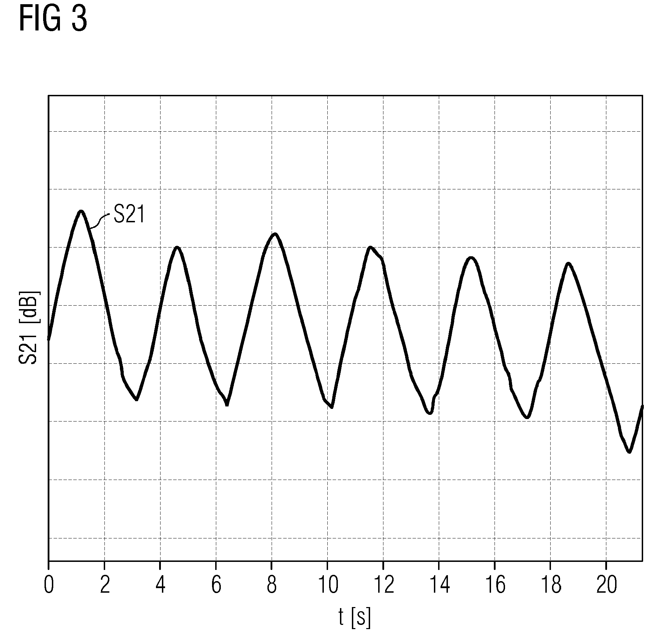

Ferner können Atembewegungen AB des Patienten 105 als in Fig. 3 gezeigte, zeitliche Änderungen S21-diff der Transmission S21-e - S21-a (vor und nach dem Einatmen) im Laufe der Zeit t[s] gemessen werden. Für eine MRT-Anwendung ist es günstig, wenn die Resonanzfrequenz des HF-Resonators HF-Res sich von der MR-Frequenz des Magnetresonanztomographiegeräts 101 unterscheidet, damit wenig oder möglichst keine gegenseitige Störung zwischen dem Bewegungssensor bzw. Atemsensor AS und einer MR-Bildgebung entstehen kann.

Wenn die Abmessungen des benutzten HF-Resonators HF-Res deutlich kleiner sind als die benutzte Wellenlänge des Messsignals, dann verhält sich der HF-Resonator HF-Res z.B. als ein induktiver oder kapazitiver Sensor und nicht (oder kaum) wie eine MRT-Bildgebungs-Antenne (106), weil man im induktiven (bzw. kapazitiven) Nahfeld arbeitet. Dadurch wird die abgestrahlte HF-Energie minimiert.

Ausgestaltungen der Erfindung können sensibel für die Atmung sein, weil die gemessenen Transmissionsverluste eines HF-Resonators HF-Res von der Luft-Gewebe-Verteilung abhängig sind; das Muskel-Gewebe kann große HF-Verluste im Vergleich mit der eingeatmeten Luft haben.

Eine Transmissionsmessung benötigt keinen Richtkoppler wie eine Messung des Reflexionsfaktors und kann weniger empfindlich auf Fehlanpassungen in einer Messstrecke sein. Erfindungsgemäße Transmissionsmessungen sind in eine großen Dynamikbereich durchführbar und können weniger mehrdeutig (Nulldurchgang und Vorzeichenänderung) als eine Messung des Reflexionsfaktors sein.

Ein HF-Resonator HF-Res kann ähnlich wie ein induktiver Sensor mit einer vernachlässigbaren Abstrahlung arbeiten, insoweit nur das Nahfeld für die Funktion notwendig ist.

Eine berührungslose Messung kann ermöglicht werden. Der Atemsensor muss nicht auf dem Körper eines Patienten 105 platziert und befestigt werden.

Ausgestaltungen der Erfindung können einen vernachlässigbaren Einfluss auf die SAR-Bilanz haben (im Gegensatz zu MR-Navigatoren) haben, insoweit die benutzten HF-Signale eine geringe Leistung haben kann (von z.B. 1mW).

Ein Bewegungssensor AS kann in einer Patientenliege 104 integriert sein. Derart kann der Körper-Abstand immer gleich sein und im Vergleich mit anderen Methoden keine neue sichtbare Verkabelung notwendig werden. Der Abstand zwischen einem Bewegungssensor AS und einem Patient 105 kann bei einem in einer Patientenliege 104 integrierten Bewegungssensor AS sehr konstant sein, insbesondere im Vergleich zu der Alternative, den Sensor oberhalb des Patienten hinter der Bore-Verkleidung zu platzieren.

Wie eine Transmissionsmessung ausführbar sein könnte, ist dem Fachmann an sich bekannt z.B. auch aus www.wikipedia.de; im Prinzip werden z.B. S-Parameter wie z.B. der Vorwärts-Transmissionsfaktor S21 mit Hilfe von Netzwerkanalysatoren als Funktion der Frequenz gemessen, s. z.B. in http://de.wikipedia.org/wiki/Netzwerkanalysator#mediaviewer/F ile:Vna3.png dargestellte Netzwerkanalysatoren, es können aber auch beliebige andere Netzwerkanalanalysatoren erwogen werden.

In Fig. 1 ist z.B. allgemein ein HF-Filter HF-Filt und ein HF-Detektor HF-Detect zur Detektion eines HF-Signals dargestellt.

## Patentansprüche

1. Bewegungssensor (AS) zur Detektion von Bewegungen (AB) eines Patienten (105) in einem bildgebenden medizinischen System, insbesondere in einem Magnetresonanztomographiesystem (101),
**dadurch gekennzeichnet,**
**dass** er (AS) mindestens einen HF-Resonator (HF-Res) zum Senden eines von einer HF-Signal-Quelle (HF-Si-Srs) in ihn eingespeisten (CPL-in) HF-Signals (HF-Si)
und zum Empfang (CPL-out, HF-Filt, HF-Dtect) des Signals (HF-Si) aufweist,
und **dass** er (AS) eine Detektionseinrichtung (HF-Filt, HF-Detect) zur Detektion von Bewegungen (AB) des Patienten (105) aufweist.

2. Bewegungssensor nach Anspruch 1,
**dadurch gekennzeichnet,**
**dass** sich die Frequenz des vom HF-Resonator (HF-Res) sendbaren HF-Signals (HF-Si) sich von der Frequenz von sendbaren Hochfrequenz-Anregungspulsen B1(x, y, z, t) des medizinischen System in Form eines Magnetresonanztomographiegeräts (101, 108) unterscheidet.

3. Bewegungssensor nach einem der vorhergehenden Ansprüche,
**dadurch gekennzeichnet, dass**
genau einer oder mehrere HF-Resonatoren (HF-Res) vorgesehen sind.

4. Bewegungssensor nach einem der vorhergehenden Ansprüche, **dadurch gekennzeichnet, dass**
der HF-Resonator (HF-Res) einen LC-Resonator mit mindestens einer Spule (LR) und mindestens einem Kondensator (CR) aufweist.

5. Bewegungssensor nach einem der vorhergehenden Ansprüche,
**dadurch gekennzeichnet, dass**
ein vom HF-Resonator (HF-Res) empfangenes HF-Signal (HF-Si) mit einem vorzugsweise schmalbandigen HF-Detektor (HF-Filt, HF-Dtect) detektiert wird.

6. Bewegungssensor nach einem der vorhergehenden Ansprüche,
**dadurch gekennzeichnet, dass**
Unterschiede (S21diff) des detektierten HF-Signals (HF-Si) im eingeatmeten und ausgeatmeten Zustand des Patienten gemessen werden.

7. Bewegungssensor nach einem der vorhergehenden Ansprüche,
**dadurch gekennzeichnet, dass**
der HF-Resonator (HF-Res) so aufgebaut ist, dass das induzierte elektromagnetische Feld (HF-Si) des HF-Resonators (HF-Res) in den Körper des Patienten (105) eindringt, damit eine Wechselwirkung (WW) zwischen dem Resonator (HF-Res) und dem Körper des Patienten (105) ermöglicht wird.

8. Bewegungssensor nach einem der vorhergehenden Ansprüche,
**dadurch gekennzeichnet, dass**
mindestens ein HF-Resonator (HF-Res) in der Nähe und/oder unterhalb des Brust-Bereichs (BrB) und/oder Bauch-Bereichs (BaB) eines Patienten (105) angeordnet ist.

9. Bewegungssensor nach einem der vorhergehenden Ansprüche,
**dadurch gekennzeichnet, dass**
der Bewegungssensor (AS) in einer Patientenliege (104) eines Magnetresonanztomographiesystems (101) angeordnet ist.

10. Bewegungssensor nach einem der vorhergehenden Ansprüche,
**dadurch gekennzeichnet, dass**
ein HF-Resonator (HF-Res) oberhalb des Patienten hinter der Bore-Verkleidung des Magnetresonanztomographiesystems (101) angeordnet ist.

11. Bewegungssensor nach einem der vorhergehenden Ansprüche,
**dadurch gekennzeichnet, dass**
er dazu ausgebildet ist, eine Körperbewegung und/oder eine Verschiebung der Anatomie innerhalb des Körpers eines Patienten (105) durch eine Änderung der belasteten Güte des HF-Resonators (HF-Res) und/oder eines Transmissionsfaktors (S21) zu detektieren.

12. Bewegungssensor nach einem der vorhergehenden Ansprüche,
**dadurch gekennzeichnet, dass**
er dazu ausgebildet (119) ist, eine Körperbewegung oder eine Verschiebung der Anatomie innerhalb des Körpers eines Patienten (105) aufgrund einer zeitlichen Änderungen der Transmission (S21) zu detektieren. (Fig. 3)

13. Bewegungssensor nach einem der vorhergehenden Ansprüche,
**dadurch gekennzeichnet, dass**
die Abmessungen des HF-Resonators (HF-Res) kleiner sind als die Wellenlänge des HF-Signals (HF-Si).

14. Bewegungssensor nach einem der vorhergehenden Ansprüche,
**dadurch gekennzeichnet, dass**
der HF-Resonator (HF-Res) dazu ausgebildet ist, als ein induktiver und/oder kapazitiver Sensor im induktiven und/oder kapazitiven Nahfeld zu arbeiten.

15. Bewegungssensor nach einem der vorhergehenden Ansprüche,
**dadurch gekennzeichnet, dass**
er dazu ausgebildet ist, eine Atmungsbewegung eines Patienten (105) eines MRT (101) berührungslos zu messen.

16. Bewegungssensor nach einem der vorhergehenden Ansprüche,
**dadurch gekennzeichnet, dass**
die benutzten HF-Signale eine Leistung von weniger als 10 mW, insbesondere maximal 1mW haben.

17. Bewegungssensor nach einem der vorhergehenden Ansprüche,
**dadurch gekennzeichnet, dass**
er ein Atemsensor zur Erfassung von Atembewegungen eines Patienten (105) in einem MRT (101) ist.

18. Bewegungssensor nach einem der vorhergehenden Ansprüche,
**dadurch gekennzeichnet, dass**
er mit einem Magnetresonanztomographiesystem (101) verbunden ist, das dazu ausgebildet (119) ist, eine aufgrund einer zeitlichen Änderungen der Transmission (S21) detektierte gemessene Körperbewegung und/oder Verschiebung der Anatomie innerhalb des Körpers eines Patienten (105) bei einer Korrektur von mit dem Magnetresonanztomographiesystem (101) bestimmten Bilddaten zur Erzeugung von Bildern des Körpers des Patienten (105) zu berücksichtigen.

19. Bewegungssensor nach einem der vorhergehenden Ansprüche,
**dadurch gekennzeichnet, dass**
das bildgebende medizinische System ein Magnetresonanztomographiesystem (101) ist.

20. Bewegungssensor nach einem der vorhergehenden Ansprüche,
**dadurch gekennzeichnet, dass** die Einkoppelung des HF-Signals (HF-Si) in den HF-Resonator (HF-Res) und/oder die Auskoppelung des HF-Signals (HF-Si) aus dem HF-Resonator
induktiv (Cpl-in, Cpl-out) oder kapazitiv oder mit einer andere Koppelung vorgesehen ist.

21. Verfahren zur Erfassung von Bewegungen (AB) eines Patienten (105) in einem bildgebenden medizinischen System, insbesondere in einem Magnetresonanztomographiesystem (101),
**dadurch gekennzeichnet, dass** ein Bewegungssensor (AS) nach einem der vorhergehenden Ansprüche verwendet wird.
